# EUROPEAN PATENT SPECIFICATION

(11) **EP 3 173 010 B1**
(45) Date of publication and mention of the grant of the patent: **15.05.2019**
(21) Application number: 15196319.6
(22) Date of filing: 25.11.2015
(51) Int. Cl.: A61B 1/04

(54) **PASSIVE CAPSULE TYPE ENDOSCOPE FOR THE INTESTINE**
PASSIVES KAPSELENDOSKOP FÜR DEN DARM
ENDOSCOPE DE TYPE CAPSULE PASSIVE POUR L'INTESTIN

(43) Date of publication of application: 31.05.2017
(73) Proprietor: Ovesco Endoscopy AG, 72074 Tübingen (DE)
(72) Inventor: Schostek, Sebastian, Dr., 72074 Tübingen (DE); Schurr, Marc O., Prof. Dr. med., 72074 Tübingen (DE)
(74) Representative: Winter, Brandl, Fürniss, Hübner, Röss, Kaiser, Polte - Partnerschaft mbB

(56) References cited:
- US-A1- 2002 198 439
- US-A1- 2005 065 441
- US-A1- 2008 242 931
- US-A1- 2014 005 758

## Description

The present invention relates to a device for imaging body cavities or passages, in particular to a passive capsule type endoscope for the small intestine, taking pictures from the inside of said small intestine and storing or transmitting these pictures to a storage device and/or a processing device.

Devices for imaging body cavities or passages in vivo are known in the art and include endoscopes and autonomous encapsulated cameras. Endoscopes are flexible or rigid tubes that pass into the body through an orifice or surgical opening, typically into the esophagus via the mouth or into the colon via the rectum.

Because of the difficulty traversing a convoluted passage, conventional flexible endoscopes cannot reach the majority of the small intestine and special techniques and precautions, that add cost, are required to reach the entirety of the colon. Endoscopic risks include possible perforation of the bodily organs traversed and complications arising from anaesthesia. Moreover, a trade-off must be made between patient pain during the procedure and the health risks and post-procedural down time associated with sedation and analgesia. Endoscopies are specialized medical services that involve a significant amount of time from clinicians and thus are costly.

An alternative in vivo image sensor that addresses many of these problems is a capsule endoscope. A camera is housed in a swallowable capsule, often along with a radio transmitter for transmitting data. The data primarily comprising images recorded by a digital camera and are transmitted to a base-station receiver or transceiver and data recorder outside the body. The capsule may also include a radio receiver for receiving instructions or other data from a base-station transmitter. Instead of radiofrequency transmission, lower-frequency electromagnetic signals may be used. Power may be supplied inductively from an external inductor to an internal inductor within the capsule or from a battery within the capsule. Parallel to the possibility to transmit the image data, capsule endoscopes exist, which have a storage unit/memory (RAM) within the capsule (internal memory). The pictures recorded by the digital camera are stored on the internal memory/storage. After the capsule has left the body through a natural orifice, the capsule is opened and the pictures on the storage are transmitted over a wireless or wired connection to, e.g. a PC or another device which can store and/or process these data.

Hereinafter, also concerning the invention, spaces which are inspected by a capsule type endoscope are perceived as "examination space". In the case of medical applications these are understood to be hollow organs or hollow spaces in the human or animal body into which a capsule type endoscope can be introduced. The terms of endoscope capsule or capsule type endoscope or capsule endoscope are equivalent in this description.

Capsule type endoscopes for inspecting an examination space like the small intestinal tract which are presently available on the market, are swallowed by a patient like a pill or a capsule and are moved passively inside the body by the peristaltic and the body motion. The camera integrated in the capsule therefore takes random pictures of the organ wall/surface of the tissue, triggered by a given imaging frequency, programmed in the capsule. But capsule endoscopes currently build and sold on the market produce a big amount of pictures/picture data. Depending on how long the endoscope lasts in the body, how many cameras the capsule has (some have more than one to achieve a better view) and how many frames/pictures per second the cameras take, up to 100.000 images and even more are produced, which results in an immense effort for the people (doctors, clinic staff) to analyze these pictures. In addition, the time- not motion-triggered imaging of conventional capsules can also lead to missing areas of interest if the capsule moves quickly forward and takes only a few pictures of a pathological area during that movement.

The document US 7983458 B2 discloses a capsule camera apparatus with a swallowable housing. The housing accommodates a light source, a camera for capturing a first and a second digital image of a scene illuminated by the light source, a motion detector for detecting a motion of the housing and an archival storage for storing the captured images.

The motion detection of the capsule is conducted using a portion of each image, the portion being stored in a partial frame buffer. In one embodiment of the US 7983458 B2, two partial frame buffers are used as an operand partial frame buffer and a reference frame buffer, respectively. The reference frame buffer is the one containing a previously stored or transmitted digital image. When the image in the operand partial frame buffer is determined not to be stored, that partial frame buffer may be overwritten by the next digital image to be motion-detected. Otherwise, the operand partial frame buffer would be designated the next reference partial frame buffer, and the current reference partial frame buffer is designated the next operand partial frame buffer. Another way to detect motion is performed by computing, motion vectors, absolute differences between the digital images, or by comparing "centers-of-mass" of the images.

The benefits of selecting captured images, based on whether the capsule has moved over a meaningful distance or orientation, is also applicable to select captured images for wireless transmission. In this manner, an image that does not provide additional information than the previously transmitted one is not transmitted. Battery power that would otherwise be required to transmit the image is therefore saved.

The EP 1039830 B1 discloses an energy saving device for acquiring in vivo images of the gastro-intestinal tract. The device, such as an autonomous capsule, includes at least one imaging unit, a control unit connected to the imaging unit, and a power supply connected to the control unit. The control unit includes a switching unit and an axial motion detector connected to the switching unit. The axial motion detector detects the axial movement of the device and if the axial acceleration is below a pre-determined threshold, disconnects the power supply thereby preventing the acquisition of redundant images.

In the US 8,005,279 a capsule endoscope image display controller is disclosed, with a video state classifying unit that can classify, for each image included in the image sequence, a video state of the image. With this unit, a "capsule moving state" can be detected indicating that the capsule endoscope is moving, based on the similarity and the amount of movement of the image.

The WO 2007/062 404 A2 discloses an endoscope capsule where the pictures are transmitted to an outside storage. A data compression within the capsule based on motion detection is used. This means, that only a change in the content of two pictures is transmitted. A change in the content between two pictures means, that the capsule has moved. In this way, only changes between two consecutive pictures have to be transmitted to an outside data storage or PC. The storage of redundant data and pictures is avoided.

The US 2004/0258328 A1 provides a device, system and method for calculation of object size by conversion of two-dimensional images, where the two-dimensional images are acquired by a moving imager. A distance-detecting unit for determining a travelled distance by the moving imager (endoscope capsule) during the capture of two of the images, is provided and at least one processor for generating spatial coordinates of objects within the images. The processor uses the distance obtained by the distance-detecting unit, and converts the spatial coordinates into a size calculation of the object.

The DE 69837160 T2 discloses a capsule endoscope with an axial motion sensor, an axial accelerometer. If an axial movement is detected, a switch connects or disconnects the power supply to the devices in the capsule. This has the effect that pictures can only be taken, if an axial movement of the capsule is detected. Additionally, false alarms, arising from body movements having a component in the axial direction of the capsule could also actuate an otherwise dormant capsule, if the signal amplitude is above a predefined threshold occurs. In order to detect such body movements an external detector can be employed in addition to the internal accelerometer of the capsule.
The website: http://www.givenimaging.com/en-int/Innovative-Solutions/Capsule-Endoscopy/Pillcam-SB/PillCam-SB-3/Pages/default.aspx from "Given Imaging" discloses the features of a current endoscopic capsule model SB 3. An adaptive frame rate technology is disclosed which allows the system to sense when the capsule is moving quickly and automatically increases the image capture rate from 2 to 6 frames per second.

Further prior art can be found in US 2008/0242931 A1 which shows an endoscopic capsule that takes pictures at a fixed frame rate or interval during examination of a patient and upon reaching a target region, said interval will be set to be shorter. Location detection therein functions via acceleration sensor and integrator or via electrical field detection or via operation time measurement.

The swallowable capsule shown in US 2014/0005758 A1 is used for applying phototherapy to a specific target site in an GI tract, wherein a controller activates a therapeutic light source based, at least in part, on a determined speed for optimal exposure time of tissue to be treated. Therein, several methods are disclosed for determining the reaching of the target site, i.e. location based activation where the location is determined via accelerations sensor and integrator or via simple photodetectors.

US 2002/0198439 A1 discloses an endoscopic capsule wherein a camera and an illuminating element are triggered upon reaching a target region or a frame rate is adjusted, wherein the reaching of the target region is determined via an external plate as a marker and electromagnetic field sensors.

US 2005/0065441 A1 describes a system, apparatus and method for measuring motion parameters concerning movement, distance, velocity, etc. of an in-vivo device utilizing motion illumination sources and motion illumination detectors to measure reflections due to for example surface or flow irregularities.

Generally spoken, in some of the current capsule endoscopes on the market it is tried to detect a motion of the capsule. The motion detection is mostly used to perform a compression of the image data and to avoid storing redundant information/pictures for saving time in the analysis of the pictures and/or to save storage space.

To detect a motion of the capsule, the prior art in principle uses two methods: In a first method, the camera in the capsule endoscope takes pictures and the content of one picture is compared to the next (or previous) picture. If a significant change is stated, this signalizes, that the capsule has moved. This method is based on the data compression methods used in video technology (mpeg, AVC etc). The benefit from this is that only changes in the content of the pictures have to be transmitted or stored. This can reduce, for example, power consumption, since the amount of data to be transmitted, is significantly reduced. Additionally, the time used for evaluating the pictures is reduced, due to the increased redundancy. The camera frame rate can be adjusted suitably, depending on the detected amount of motion.

A second method in prior art capsules performs a movement detection with the help of a motion sensor which is accommodated in the capsule. The detection takes place in the direction of the longitudinal axis (which is more or less parallel to the axis of the small intestine). Only when a movement is detected, camera and illumination in the capsule are activated. But with these sensors it is not detectable, how far exactly the capsule has moved in the small intestine. Therefore, this method simply prevents the camera from taking pictures if the camera or the capsule is not in motion. But this does not necessarily reduce a redundancy of the acquired pictures.

What is needed in the art is an endoscope capsule which can detect a movement of the capsule, relative to the examination space (here: the small intestine) which is examined. Thereby activating the camera in fixed, evenly, adjustable intervals. This preventing the capsule from simply taking time triggered "random" pictures. Moreover a capsule is needed, which can exactly detect a distance, the capsule has travelled in the examination space. The advantage of such an endoscopic capsule is, that the power consumption of the capsule is by far lesser than in prior art, the number of the pictures is increased due to lack of unnecessary redundancy and the time needed for the analysis of the pictures is reduced.

This objective is achieved by a capsule type endoscope comprising the features of claim 1. Advantageous further developments of the invention are the subject matter of the subclaims.

In the following, the term "image recording unit" may also be referred to as a "camera", a "digital camera" or equivalents thereof.

In a preferred embodiment, the endoscope capsule for examining a body cavity comprises means for acquiring images or an imaging unit and means for the detection of movement or a motion detector and means for processing data or a data processing unit, whereby the data processing unit is configured and intended to activate the imaging unit to perform imaging recording dependent of a distance the endoscope capsule has covered, determined by the means for the detection of movement.

According to another embodiment of the present invention, an autonomous video capsule or endoscope capsule for acquiring a sequence of images within an examination space is provided, comprising a capsule like housing with an imaging unit or means for acquiring images that records single images or a series of single images (video) of an examination space. The imaging unit is accommodated in the capsule and arranged on an end of the capsule. A transparent end portion can be arranged on one end of the capsule. The transparent portion can, in another embodiment, cover the imaging unit (imaging means) or a lens of the imaging unit. The video capsule or endoscope capsule can further comprise means for the detection of movement or a motion detector for detecting a motion of said capsule. Preferably, the movement is detected relative to a tissue surface of the examination space. The capsule can further comprise processing and storage means within the capsule. At least one illumination means or light source can be provided within the capsule and an energy source within the capsule for the energy supply of the imaging unit, the motion detector, the processing and storage means and the light source, wherein the motion detector is configured to detect a distance which the capsule has covered alongside the tissue surface of the examination space and is further configured to activate the imaging unit and the light source to record pictures/images of the examination space when the capsule has covered a predetermined distance in the examination space after a previously recorded picture.

Preferably, the front-camera or means for acquiring images is in a kind of "sleep-mode" if not in use. The camera is optimized for low power consumption to save battery power. The front camera has an optical axis. Preferably, the optical axis of the front camera is parallel to the direction in which the capsule moves in the hollow organ.

The camera is preferably activated by an external signal. The external signal is preferably generated by the motion detector but can also be generated from outside the body.

The illumination is activated preferably synchronously with the camera. Similar to the activation of the camera, the signal for the activation of the illumination is done by an external signal. The external signal is preferably generated by the motion detector but can also be generated from outside the body.

Alternatively, the activation signal for the illumination is generated by the camera chip.

Preferably, the camera has a resolution of 640x640 pixels. The camera chip preferably takes color images. The resolution of the camera can be variable. According to the requirements during an examination, the resolution may be scalable. For example, the resolution can be scaled down to 320x320 pixel or 160x160 pixels. It is emphasized, that these resolution are just exemplary and do not serve to limit the invention. Future camera-chips may have different resolutions which may be higher and may also have different picture proportions (length-to-wide).

The camera has optic/lenses which allow the camera system of the capsule to cover a field of vision of 5 to 15 mm, if the capsule is used to inspect the small intestine. If, for example, the examination space is the colon or the stomach, the field of view is extendable from 5 to 100mm. These values for the field of view are not intended to limit the scope of the present invention. For different fields of application, the field of view of the front camera in the capsule may be chosen appropriately. The lenses/optics are covered by the transparent cover.

Alternatively, the transparent cover may be configured and made of a material to serve as the lens/optics. An extra lens/optics is therefore not needed which saves cost.

The camera-system preferably contains an LVDS interface to transmit the image data. LVDS is a differential signalling system, meaning that it transmits information as a difference between the voltages on a pair of wires. This does not limit the scope of the invention and other modes of data-transmission between the different systems in the capsule are possible.

The camera is optimized for a high signal to noise ratio (SNR) to enable an optimized picture quality at low illumination. This results in saving battery power, since the illumination, which is one of the devices with the highest energy consumption, can be operated at a low and energy saving level.

The processing and storage means within the capsule may be separate circuits. Alternatively, a circuit with integrated memory and processing means is imaginable as an ASIC. The storage/memory can be read wirelessly (e.g. Bluetooth or WLAN) or via contacts (e.g. a USB-connection) which are accessible from outside the capsule. The storage/memory may be a separate chip (e.g. micro SD) which can be removed and read out after the capsule has left the body and was opened.

In a preferred embodiment of the capsule type endoscope the motion detector is situated within the capsule endoscope. Alternatively, the motion detector is an external device, not accommodated in the capsule, which can activate the camera and/or the illumination of the camera in the capsule with an appropriate signal. The external motion detector is configured to exactly detect a motion of the capsule relative to the tissue surface, the capsule is surrounded with.

In another preferred embodiment of the capsule type endoscope, the motion detector is an optical motion detector. Another expression for the optical motion detector is "optical tracer". Preferably, the optical motion detector/optical tracer has a working principle similar to an optical mouse. An optical mouse has a small, light-emitting diode (LED) that bounces light off a surface to be scanned and from that surface, the light bounces back onto an optical complementary metal-oxide semiconductor (CMOS) sensor. The CMOS sensor sends each captured image of the surface image to a digital signal processor (DSP) for analysis. The CMOS sensor of the motion detector/optical tracer actually can therefore be considered as a tiny camera and is configured to take up to 60 pictures or more every second. It is able to work on almost any surface on which a movement can be detected. The surface of a body tissue in an examination space is suitable for that purpose. The DSP in the motion detector/tracer is fast enough to detect patterns in the images (on the surfaces respectively) and can evaluate how those patterns have changed since the previous image. Based on the change in patterns over a sequence of images, the DSP determines if or how far the capsule has moved. An exact distance of the movement can be detected which is used as a trigger for the front-camera.

The optical motion detector and its optical system, in particular its illumination (LED) and its optical sensor is different to the optical system and the illumination unit of the imaging unit. That is, the illumination of the camera/imaging unit is different to the illumination of the motion detector and the front-camera/imaging system of the capsule is different to camera-chip/optical sensor (CMOS-sensor) of the motion detector. The optical system of the optical motion detector has an optical axis.

The area which the optical sensor of the motion detector scans is preferably between 0,125x0,125 mm² and 1x1 mm². Preferably, the resolution of the small camera (the CMOS sensor respectively) in the motion detector is up to 256x256 pixels. This resolution corresponds to a resolution of 6500dpi and matches with currently available sensors on the market. This resolution is not considered to limit the scope of the invention. The use of sensors with different resolution sizes is possible according to examination needs.

The frames per second rate of the CMOS sensor of the motion detector, meaning the number of "pictures" the sensor takes from the tissue surface in the examination space, is preferably between 30 fps - 60 fps for this purpose. If necessary, other frame rates may be used adapted to specific examination necessities (e.g. the speed, the capsule proceeds in the examination space).

The motion detector/tracer preferably uses a monochromatic sensor. The illumination colour of the sensor light is monochromatic as well. If necessary, an optimal light colour of the light source in the motion detector is evaluated. The motion detection eventually can be improved with an optimal illumination. In other words, dependent on the examination needs, different type of endoscopic capsules are available which work according the same principle, but differ regarding resolution of the CMOS sensor of the motion detector and the colour of the lighting of the motion detector.

Preferably, the motion detector uses an LVDS interface for the data-communication with the internal electronic modules.

The focus of the optical system of the motion detector/tracer is preferably aligned with the capsule surface or the tissue surface. Preferably, the tissue surface is in contact with the capsule surface. In other words, the plane, in which the light source of the motion detector is focused, is the tissue surface.

In a preferred embodiment, the capsule type endoscope is configured in a way, that it can specifically examine an examination space which is a small intestine. That means, the capsule has a size that the surface of the capsule is in physical contact with the tissue surface of the small intestine.

In yet another embodiment of the capsule type endoscope, a focal plane of the motion detector is aligned with the tissue surface. In the small intestine, the diameter of the small intestine is smaller than the diameter of the capsule endoscope. Therefore, the capsule surface is in contact with the tissue surface and it is justified to say that in a small intestine the focal plane is aligned with the tissue surface. In other words, for an examination in which the tissue surface is in contact with the surface of the capsule, the focal plane or the focus of the motion sensor is preferably a fixed focus.

The alignment with the tissue surface is currently needed for the optical motion detection to detect a movement of the capsule. Nevertheless, alternatively other optical motion sensors are imaginable which can exactly detect a change and a distance of movement of an endoscopic capsule relative to a surface in an examination space and where it is not necessary that a focal plane is aligned with the capsule/tissue surface.

In yet another embodiment of the capsule type endoscope, the endoscope comprises an internal storage unit or memory on which the pictures/images of the examination area/small intestine can be stored. The storage unit may be a removable storage unit which can be removed from the capsule after the capsule has left the body. Alternatively, the capsule endoscope is adapted to read the stored image data wirelessly, without removing the memory. In another alternative, the capsule may have a connector to read-out the stored image data. The connector may be accessible inside the capsule, after opening it or on the outside of the capsule in form of metallic contacts. The memory can be any type of non-volatile memory available on the market.

The memory size is dependent on the camera resolution, the number of pictures and if the pictures are subjected to a data-compression/-reduction. A preferred size of memory is currently between 8 and 16 GB (gigabytes). Since the development on this technical area is proceeding very fast, said preferred memory size is not intended to limit the invention and is chosen appropriately. For example, a front-camera with a higher resolution needs a bigger memory. For the internal data transmission between camera and memory, the capsule electronic uses an LVDS interface.

An energy source in the capsule is preferably a battery in form of one or more button cells. The energy source supplies power to all systems in the capsule. Form and size of the energy source depend on the configuration of the capsule. As an alternative, the energy source may also be a device including coils adapted to launch energy by electro-magnetic induction. In this case, the capsule has to be equipped with a specific coil. Such systems are already known in prior art and therefore will not be described in detail hereinafter.

As pointed out in the foregoing, the crucial component of the capsule type endoscope is the motion detector which is adapted to and arranged this way, that it can recognize the movement of the capsule in relation to the surrounding tissue of the intestine. The distance which the capsule has traveled along the tissue surface can be determined exactly and, based on that, the camera and the illumination of the capsule can be activated in predetermined, fixed intervals or distances, to achieve an evenly image sequence. In other words, the camera in the front of the capsule only records an image, when the motion detector has stated, that the capsule has covered a specific distance since the previous picture has been recorded.

The distance between two pictures/images relative to the tissue surface is dependent on the resolution and the angle of view of the front camera in the capsule. The smaller the angle of view of the front camera is the more pictures have to be acquired, what means, the distance between consecutive pictures becomes shorter. Vice versa, if the angle of view becomes larger, less pictures/images are necessary to get a complete, continuous picture sequence of the examination space.

Preferably the capsule can, with the internal electronic subsystems and control and processing means, calculate and set the distance between consecutive pictures at which an image is taken. Alternatively, the distance is predetermined and programmed in each capsule prior to an examination.

Preferably, two consecutive images share a redundant part, so that a stitching of the images is possible during processing the pictures. The corresponding (redundant) part in the images then shows, where one image ends and the next image starts. For example: an upper part of a first picture may be the lower part of a second consecutive picture. An upper part of the second picture is the lower part of a third picture and so on. In this manner, a picture sequence showing the complete examination area containing only necessary images, is achievable.

Hereinafter the invention will be illustrated by way of a preferred embodiment with reference to the accompanying drawings, in which:
Fig. 1 shows an endoscope for inspecting the small intestine according to the state of the art.
Fig. 2 shows a capsule endoscope for inspecting the small intestine according to an embodiment of the current invention.
Fig. 3 shows a sectional view of the front of an embodiment of the capsule endoscope.
Fig. 4 shows, as a block diagram, the motion detection system and the endoscopic system inside the capsule and their interconnection.
Fig. 5 schematically shows the configuration of the motion detector.
Fig. 6 shows an enlarged view of the motion detection system of the capsule type endoscope.

### DESCRIPTION OF FIGURES

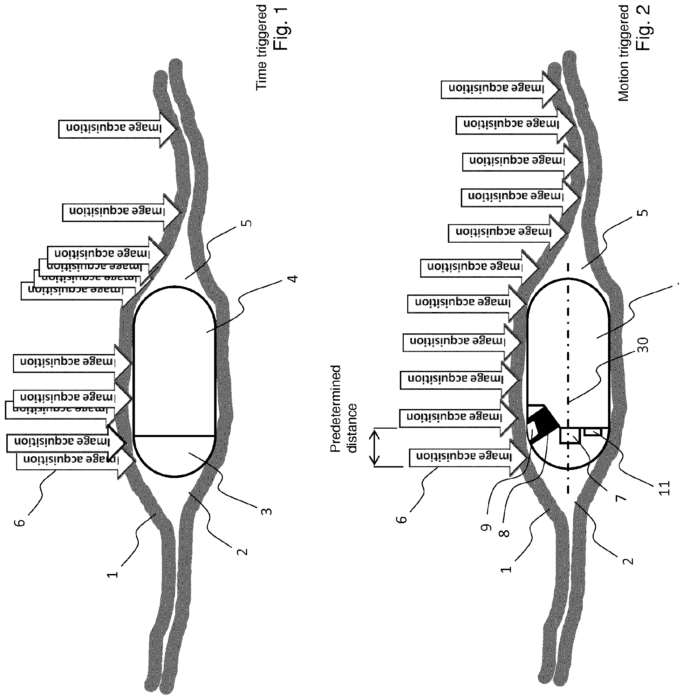

As can be seen, figure 1 shows an endoscope capsule 4 according to the prior art for an examination of the small intestine 5. The diameter of the small intestine 5 is lesser than the diameter of the capsule 4. The surface 2 of the tissue 1 to be inspected more or less completely encloses the endoscope capsule 4. The arrows 6, drawn along the way the capsule 4 moves in the small intestine 5, symbolize single pictures recorded from a digital camera (not shown here) in the front of the capsule 4, covered by a transparent cover 3. The arrows 6, respectively the pictures they symbolize, are not evenly distributed along the way in the small intestine 5. This is due to the fact, that the state of the art capsules release the camera in fixed time intervals or at least with a minimum frame rate, even if the capsule 4 is stuck in the examination space 5. If the capsule 4 does not move, the capsule 4 takes a lot of pictures of the same area, which is represented in fig. 1 by the density of the arrows. This prior art capsule detects if the capsule moves, but simply adjusts the frame-rate in case of no movement to at least a minimum frame-rate (e.g. 2fps).

Fig. 2 shows a capsule 4 according to the present invention. The capsule 4, like in fig. 1, is in the examination space, in this case the small intestine 5. The capsule 4 is surrounded by the tissue 1. The surface 2 of the tissue is in contact with the surface of the capsule 4. A motion detector 8 is arranged this way, that the motion detector 8 is also in contact with the tissue surface 2. The motion detector 8 in the capsule 4 scans the tissue surface of the examination space/small intestine 5. If the capsule does not move relative to the tissue surface 2, a sensor (not shown) in the motion detector 8 "sees" the same part of the tissue surface 2. In case, the capsule 4 is moved forward by the peristaltic motion of the body, the sensor in the motion detector 8 "sees" a different part of the tissue surface 2 and activates camera 7 and illumination 11. A picture of the examination space 5 is recorded and stored internally in the capsule 4 on a storage device (not shown). To record another picture with the camera 7, the motion detector 8 detects the distance, the capsule has travelled compared to the position the previous picture has been recorded. If a predetermined distance has covered (adjustable in the capsule), the camera 7 in the capsule 4 records another picture.

The arrows 6 in figure 2, as well as the arrows 6 in fig. 1, represent the positions, a picture has been recorded. It can be recognized, that the arrows in fig. 2 are evenly distributed what means, that the recorded pictures have a fixed predetermined distance from each other.

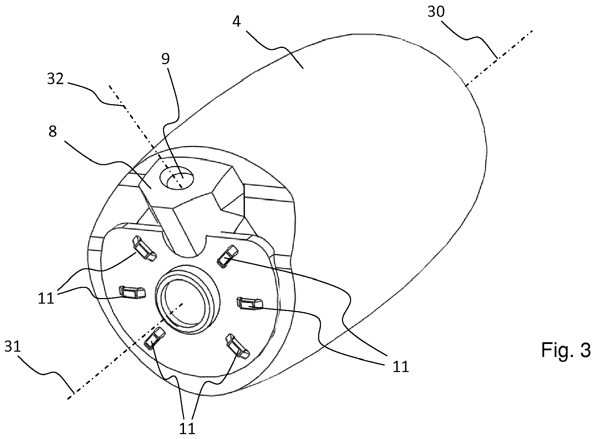

In Fig. 3, a sectional view of the front of a preferred embodiment of the capsule endoscope 4 is shown. A camera 7, in this embodiment, is arranged on a PCB, surrounded by illumination means 11 or, in other words, light sources. The camera 7 records pictures preferably in a direction parallel to the direction of movement within the examination space. The line of vision of the camera 7 is perpendicular to the plane of the PCB on which the camera 7 is arranged on in this embodiment. The illumination 11 in fig. 3 is arranged around the camera 7 in such a way, that the area, recorded by camera 7 is properly illuminated. A transparent front cover (not shown) of the capsule 4 covers the camera 7 and the light sources 11. The light sources 11 are preferably light emitting diodes (LED). The capsule has a longitudinal axis 30 along the length of the capsule geometry.

In the embodiment in fig. 3, means for detecting a movement or an optical motion detector 8 is arranged in the capsule 4. The motion detector 8 has an opening 9 that has a line of view in the direction of the tissue surface 2. The line of vision of the optical motion detector 8 is different to the line of vision of the front-camera 7 of the capsule. While the line of vision or the optical axis 31 of the front-camera 7 is essentially in line or parallel to the longitudinal axis 30 of the capsule, the line of vision or optical axis 32 of the optical motion detector 8 is not parallel to the longitudinal axis 30 of the capsule. In other words, the line of vision or optical axis 32 of the optical motion detector 8 is not parallel to the line of vision or the optical axis 31 of the front-camera 7. Preferably, the optical axis 32 of the optical motion detector 8 is essentially perpendicular to the longitudinal axis 30 of the capsule or to the optical axis 31 of the front-camera 7. The latter is not intended to restrict the present invention. Different angles between the optical axis 32 of the optical motion detector 8 and the optical axis 31 of the front-camera 7 are possible. The opening 9 is covered with a transparent cover (not shown here). The opening 9 of the motion detector 8 is preferably aligned with the surface of the capsule.

In an alternative embodiment, the front camera may be configured in a way, that it also can scan the tissue surface of the examination space and detect an exact covered distance relative to the tissue surface. In such an embodiment, a separate motion detector would not be necessary.

In another embodiment, depending on power consumption or brightness requirements, at least one light source 11 is arranged in the capsule 4 to illuminate the area of interest in the examination space. Preferably, the light source 11 or light sources emit white light. Depending on the examination requirements, other suitable colors are possible to illuminate the examination area 5.

In yet another embodiment, the motion detector 8 may be a separate part and arranged outside the capsule/body and is configured, to detect a movement of the capsule 4 and can further detect a traveled distance of the capsule relative to a tissue surface 2.

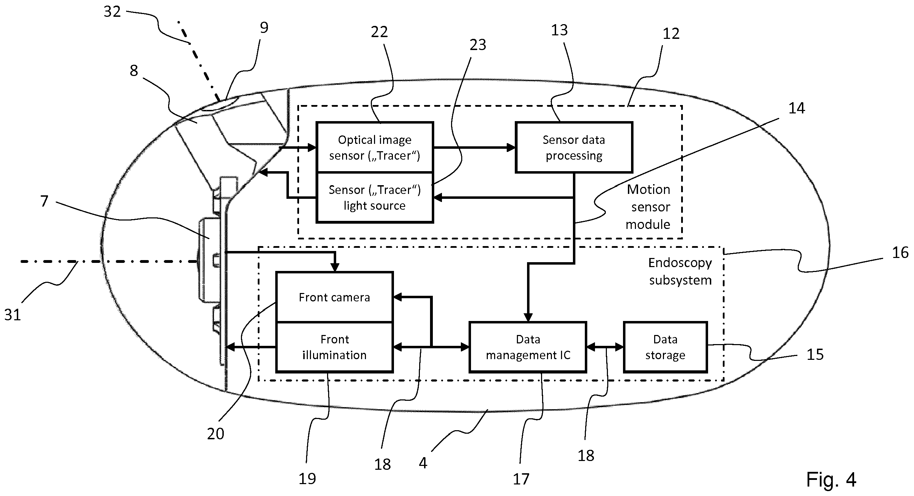

In the embodiment in Figure 4 the function blocks of a preferred embodiment of the internal data processing in the endoscopic capsule 4 is shown. Two big function blocks or subsystems are present, the motion sensor module (tracer) 12 and the endoscopic system 16.

The motion sensor module 12 has a unit 13 for processing the sensor data and is responsible for the motion detection of the capsule 4 and therefore for the processing of the data of the optical image sensor ("tracer") 22. This data processing unit 13, which is a specific chip for this purpose, is in a data communication with the optical image sensor 22 and receives data ("raw data") from the optical image sensor 22 of the tracer. The data processing unit 13 also controls the sensor light source 23 of the "tracer". Based on the received data from the optical image sensor 22 of the tracer, the unit 13 for processing the sensor data detects, if the capsule 4 moves/has moved within the examination space, or not. For a fast detection, the unit 13 uses a DSP to analyze the pictures from the camera/optical image sensor 22. Additionally, the unit 13 for processing the sensor data detects the distance the capsule travels in the examination space. The unit 13 for processing the sensor data therefore not only controls the sensor light and the tracer camera/optical image sensor 22, but also generates a result regarding movement of the capsule 4. The unit 13 for processing the sensor data is also in a data communication 14 with the second subsystem, the Endoscopy-subsystem" 16. The data submitted from the first subsystem (motion sensor module (tracer) 12) to the endoscopic subsystem 16, are processed in another circuit, the data management unit (data management IC) 17. The data management IC 17 is in data connection 18 with a data storage 15, a control unit 20 for the front camera 7 and a control unit 19 for the front illumination 11. In case, the data management IC 17 detects a movement of the capsule or in other words, the data of the motion sensor module 12 state, that the capsule moves, front camera 7 and illumination 11 are activated synchronously. The picture which has been acquired is transferred to the data management IC 17 which stores the image in the storage unit 15.

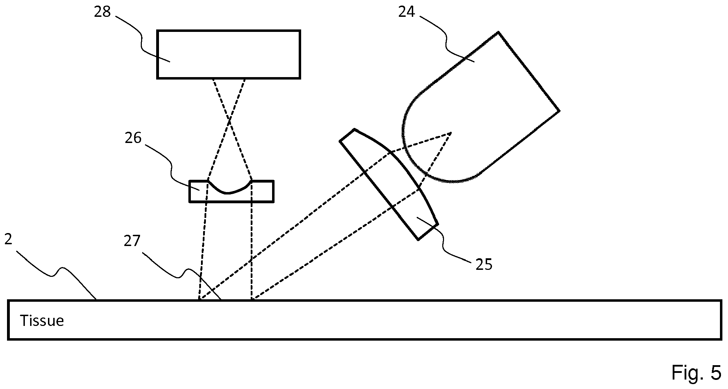

Fig. 5 is a schematic view of the principle operation of the tracer/motion detector 8. A light source 24 (LED) in the detector emits light or a light beam, which is focused by a lens 25. The lens 25 is preferably an integral part of the motion detector/tracer 8. The focused light beam hits the tissue 1, or, better said, the surface 2 of the tissue 1 in an examination space (small intestine). The plane, in which the light hits the tissue surface, can be considered as the focal-plane 27 of the system. The light is bounced back from the tissue surface to an optical sensor 18, which can be considered as a small camera. A surface has a "structure" or pattern. The light emitted by the LED illuminates a small area on the surface 2 (in this case: the surface of the tissue of the examination space/small intestine). When the light of the light source 24 of the motion detector 8 is bounced back and focused on the sensor 18 by lens 26, the sensor 18 recognizes this pattern. Since the measures (distance from LED to lens, lens to surface, measures of the illuminated area, distance to optical sensor) are known, the sensor 18 or, better said, processing means in the motion sensor module (not shown) determine, if the pattern has changed and therefore some kind of movement took place. With this information, the motion detector determines how far the capsule has moved relative to a previous "pattern" the optical image sensor 18 of the motion detector 8 has detected. Since this is checked many times per second, the traveling distance of the capsule in the examination space can be determined accurately.

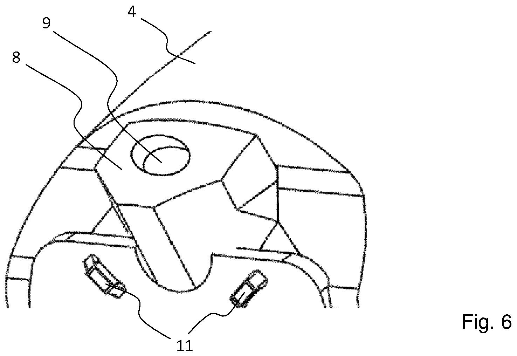

Fig. 6 shows an enlarged view of an exemplary motion detector of a preferred embodiment of the capsule endoscope. It can be recognized, that the opening 9 of the detector 8 is arranged near the surface of the capsule 4. Through opening 9, the light beams of LED 24 in fig. 5 reach the tissue surface 2 and are bounced back from there onto the sensor 18 in fig. 5. The opening 9 is preferably arranged in a way, that it is in a plane or, better said, aligned with the outer surface of the capsule 4. The opening 9 of the motion detector 8 is covered by a transparent cover (not shown). This cover is preferably an integral part of the transparent cover 3 of the capsule 4, as e.g. shown in Fig. 4. Alternatively, in another embodiment, it can be a separate transparent window in the capsule housing, independent from the transparent cover 3 of the capsule.

The opening 9 or the transparent cover of the opening 9 of the detector 8 is preferably in contact with the tissue surface 2 of the examination space 5. The focus of the optical system in the detector 8 is arranged such, that the focal plane 27 of said system is preferably aligned with the tissue surface 2 of the examination space 5 through which the capsule 4 moves. In other words, the optical motion detector 8 is arranged in a way, that it scans the tissue surface 2 to detect a movement of the capsule relative to the tissue surface 2. Since the tissue surface 2 and the surface of the capsule 4 are in contact during examination, it is justified to say that the focal plane 27 of the motion detector is on the tissue surface 2.

### Reference signs

- 1: Tissue
- 2: Tissue surface
- 3: Transparent cover
- 3a: Transparent area in cover for motion detector
- 4: Capsule/housing
- 5: Examination space/small intestine
- 6: Image acquisition
- 7: Camera, imaging unit
- 8: Motion detector
- 9: Opening of motion detector
- 10: Time interval/distance (between pictures)
- 11: Light source
- 12: Motion sensor module
- 13: unit for processing sensor data
- 14: Data connection from "motion sensor module" to "Endoscopy subsystem"
- 15: Storage/memory of capsule
- 16: endoscopy subsystem
- 17: Data management circuit
- 18: Data communication (camera/illumination to data management)
- 19: Controller front light
- 20: Controller front camera
- 21: Movement data from sensor module
- 22: Controller "optical image sensor"
- 23: Controller "sensor light source"
- 24: Sensor light source
- 25: Focus lens sensor light source (motion detector)
- 26: Focus lens sensor chip/camera (motion detector)
- 27: Focal plane of motion detector
- 28: Sensor chip (motion detector)
- 29: Energy source
- 30: Longitudinal axis of capsule
- 31: Optical axis of camera
- 32: Optical axis of

## Claims

1. Endoscope capsule for examining a body cavity containing:
an imaging unit being formed as a camera (7) comprising an optical system and an illumination unit,
means for the detection of movement (8) being formed as an optical motion detector comprising an optical sensor and an illumination being different to the optical system and illumination unit of the camera (7) and
a data processing means (12, 16) being configured to activate the camera (7) to perform imaging recording dependent on a distance the endoscope capsule has covered, which distance is determined by the optical motion detector (8), wherein
the data processing means (12, 16) comprises
an endoscopic subsystem (16) having control units (19, 20) for the optical system and the illumination unit of the camera (7) as well as a data management unit (17) being in data connection with the control units (19, 20) and
a motion sensor module or tracer (12) having a data processing unit (13) which is a specific chip for processing data of the optical motion detector (8) generating a result regarding movement of the capsule (4) and which is in data communication with the endoscopic subsystem (16) wherein
data submitted from data processing unit (13) of the motion sensor module or tracer (12) to the endoscopic subsystem (16) is processed in the data management unit (17) of the endoscopic subsystem (16).

2. The endoscope capsule according to claim 1 or 2, **characterized in that** the capsule is adapted to examine a tissue surface (2) of an examination space (5).

3. The endoscope capsule according to claim 1 or 2, **characterized in that** the optical motion detector (8) is a CMOS-sensor.

4. The endoscopic capsule according to any of the preceding claims, wherein the illumination colour of the illumination or light source of the optical motion detector (8) is monochromatic.

5. The endoscope capsule according to one any of the preceding claims2 to 4, **characterized in that** the means for the optical motion detector (8) is configured to scan the tissue surface (2) of a hollow organ (5).

6. The endoscope capsule according to claim 5, wherein an area which the optical motion detector (8) scans is preferably between 0,125 x 0,125 mm^2 and 1 x 1 mm^2.

7. The endoscope capsule according to any of the preceding claims, **characterized in that** the capsule is configured to examine the small intestine as an examination space (5).

8. The endoscope capsule according to any of the preceding claims 2 to 7, **characterized in that** the tissue surface (2) is aligned with a focal plane (27) of the means for the optical motion detector (8).

9. The endoscope capsule according to any of the preceding claims, **characterized in that** the images recorded with the camera (7) are stored on internal data storage (15) of the capsule (4).

10. The endoscope capsule according to any of the preceding claims, **characterized in that** an optical axis (31) of the camera (7) and an optical axis (32) of the optical motion detector (8) are both directed in different directions.

11. The endoscope capsule according to claim 10, wherein the optical axis (31) of the camera (7) is preferably parallel to the longitudinal axis (30) of the capsule and the optical axis (32) of the optical motion detector (8) is preferably perpendicular to the longitudinal axis (30) of the capsule.

12. The endoscope capsule according to any of the claims 3 to 11,wherein a frame rate of the optical motion detector (8) is at least 30 frames per second and preferably between 30 and 60 frames per second.

13. The endoscope capsule according to any of the preceding claims, wherein the camera (7) is activated each time the capsule has covered a predetermined, fixed distance after a previously recorded image.

14. The endoscope capsule according to any of the claims 3 to 13, wherein the motion sensor module or tracer (12) has a DSP which is adapted to detect patterns in the pictures taken by the CMOS-sensor, to evaluate how those patterns have changed since the previous picture and to determine if or how far the capsule has moved based on the change in patterns over a sequence of images.

## Patentansprüche

1. Endoskopkapsel (4) zur Untersuchung einer Körperhöhle mit:
einer Beleuchtungseinheit, die als Kamera (7) ausgebildet ist, die ein optisches System und eine Beleuchtungseinheit aufweist,
einer Vorrichtung zur Detektion einer Bewegung (8), die als ein optischer Bewegungsdetektor ausgebildet ist, der einen optischen Sensor und eine Beleuchtung aufweist, die sich von dem optischen System und der Beleuchtungseinheit der Kamera (7) unterscheiden, und
einer Datenverarbeitungsvorrichtung (12, 16), die konfiguriert ist, die Kamera (7) zu aktivieren, um eine Bildaufzeichnung in Abhängigkeit von einem Abstand durchzuführen, den die Endoskopkapsel (4) erfasst hat, wobei der Abstand durch den optischen Bewegungssensor (8) bestimmt wird, wobei
die Datenverarbeitungsvorrichtung (12, 16) ein endoskopisches Subsystem (16) aufweist, das Steuereinheiten (19, 20) für das optische System und die Beleuchtungseinheit der Kamera (7) sowie eine Datenverwaltungseinheit (17) hat, die mit den Steuereinheiten (19, 20) in Datenverbindung steht, und
ein Bewegungssensormodul oder Tracer (12) aufweist, das eine Datenverarbeitungseinheit (13) hat, die ein spezifischer Chip für die Verarbeitung der Daten des optischen Bewegungsdetektors (8) ist, der ein Ergebnis bezüglich der Bewegung der Kapsel (4) erzeugt und der in Datenübermittlung mit dem endoskopischen Subsystem (16) steht, wobei
die Daten, die von der Datenverarbeitungseinheit (13) des Bewegungssensormoduls oder Tracers (12) an das endoskopische Subsystem (16) übertragen werden, in der Datenverarbeitungseinheit (17) des endoskopischen Subsystems (16) verarbeitet werden.

2. Endoskopkapsel (4) gemäß Anspruch 1 oder 2, **dadurch gekennzeichnet, dass** die Kapsel (4) dazu angepasst ist, eine Gewebeoberfläche (2) eines Untersuchungsraumes (5) zu untersuchen.

3. Endoskopkapsel (4) gemäß Anspruch 1 oder 2, **dadurch gekennzeichnet, dass** der optische Bewegungsdetektor (8) ein CMOS-Sensor ist.

4. Endoskopkapsel (4) gemäß einem der vorangegangenen Ansprüche, wobei die Lichtfarbe der Beleuchtung oder einer Lichtquelle des optischen Bewegungsdetektors (8) monochromatisch ist.

5. Endoskopkapsel (4) gemäß einem der vorangegangenen Ansprüche 2 bis 4, **dadurch gekennzeichnet, dass** die Vorrichtung für den optischen Bewegungssensor (8) konfiguriert ist, die Gewebeoberfläche (2) eines Hohlorgans (5) zu scannen.

6. Endoskopkapsel (4) gemäß Anspruch 5, wobei eine Fläche, welche der optische Bewegungsdetektor (8) scannt, vorzugsweise zwischen 0,125x0,125mm² und 1x1 mm² liegt.

7. Endoskopkapsel (4) gemäß einem der vorangegangenen Ansprüche, **dadurch gekennzeichnet, dass** die Kapsel (4) konfiguriert ist, den Dünndarm als einen Untersuchungsraum (5) zu untersuchen.

8. Endoskopkapsel (4) gemäß einem der vorangegangenen Ansprüche 2 bis 7, **dadurch gekennzeichnet, dass** die Gewebeoberfläche (2) mit einer Fokalebene (27) der Vorrichtung für den optischen Bewegungssensor (8) ausgerichtet ist.

9. Endoskopkapsel (4) gemäß einem der vorangegangenen Ansprüche, **dadurch gekennzeichnet, dass** die Bilder, die mit der Kamera (7) aufgezeichnet werden, auf einem internen Datenspeicher (15) der Kapsel (4) gespeichert werden.

10. Endoskopkapsel (4) gemäß einem der vorangegangenen Ansprüche, **dadurch gekennzeichnet, dass** eine optische Achse (31) der Kamera (7) und eine optische Achse (32) des optischen Bewegungsdetektors (8) beide in verschiedene Richtungen gerichtet sind.

11. Endoskopkapsel (4) gemäß Anspruch 10, wobei die optische Achse (31) der Kamera (7) vorzugsweise parallel zur Längsachse (30) der Kapsel (4) ist und die optische Achse (32) des optischen Bewegungsdetektors (8) vorzugsweise senkrecht zur Längsachse (30) der Kapsel (4) ist.

12. Endoskopkapsel (4) gemäß einem der Ansprüche 3 bis 11, wobei eine Bildfrequenz des optischen Bewegungsdetektors (8) zumindest 30 Bilder pro Sekunde und vorzugsweise zwischen 30 bis 60 Bilder pro Sekunde ist.

13. Endoskopkapsel (4) gemäß einem der vorangegangenen Ansprüche, wobei die Kamera (7) jedes Mal aktiviert wird, wenn die Kapsel (4) einen vorbestimmten, fixierten Abstand zu einem zuvor aufgezeichneten Bild erfasst hat.

14. Endoskopkapsel (4) gemäß einem der Ansprüche 3 bis 13, wobei das Bewegungssensormodul oder Tracer (12) ein DSP hat, was dazu angepasst ist, Muster in den Bildern zu detektieren, die durch den CMOS-Sensor aufgenommen werden, zu evaluieren, wie sich diese Muster seit dem vorangegangen Bild verändert haben, und zu bestimmen, ob und wie weit sich die Kapsel (4) auf Grundlage der Änderung der Muster über eine Bildsequenz bewegt hat.

## Revendications

1. Capsule d'endoscope pour examiner une cavité corporelle contenant :
une unité d'imagerie étant sous la forme d'une caméra (7) comprenant un système optique et une unité d'éclairage,
un moyen de détection du mouvement (8) étant sous la forme d'un détecteur optique de mouvement comprenant un capteur optique et un éclairage étant différent du système optique et de l'unité d'éclairage de la caméra (7) et
un moyen de traitement des données (12, 16) étant configuré pour activer la caméra (7) pour effectuer un enregistrement d'imagerie en fonction d'une distance que la capsule d'endoscope a couverte, laquelle distance est déterminée par le détecteur optique de mouvement (8),
dans laquelle
le moyen de traitement des données (12, 16) comprend
un sous-système endoscopique (16) ayant des unités de commande (19, 20) pour le système optique et l'unité d'éclairage de la caméra (7) ainsi qu'une unité de gestion des données (17) étant en connexion de données avec les unités de commande (19, 20) et
un module capteur de mouvement ou traceur (12) ayant une unité de traitement des données (13) qui est une puce spécifique pour traiter les données du détecteur optique de mouvement (8) générant un résultat relatif au mouvement de la capsule (4) et qui est en communication de données avec le sous-système endoscopique (16) dans laquelle
les données soumises à partir de l'unité de traitement des données (13) du module capteur de mouvement ou traceur (12) au sous-système endoscopique (16) sont traitées dans l'unité de gestion des données (17) du sous-système endoscopique (16).

2. Capsule d'endoscope selon la revendication 1 ou 2, **caractérisée en ce que** la capsule est adaptée pour examiner une surface tissulaire (2) d'un espace sous examen (5).

3. Capsule d'endoscope selon la revendication 1 ou 2, **caractérisée en ce que** le détecteur optique de mouvement (8) est un capteur CMOS.

4. Capsule endoscopique selon l'une quelconque des revendications précédentes, dans laquelle la couleur d'éclairage de la source d'éclairage ou de lumière du détecteur optique de mouvement (8) est monochromatique.

5. Capsule d'endoscope selon l'une quelconque des revendications précédentes 2 à 4, **caractérisée en ce que** le moyen de détecteur optique de mouvement (8) est configuré pour balayer la surface tissulaire (2) d'un organe creux (5).

6. Capsule d'endoscope selon la revendication 5, dans laquelle une zone que le détecteur optique de mouvement (8) balaye est de préférence entre 0,125 x 0,125 mm^2 et 1 x 1 mm^2.

7. Capsule d'endoscope selon l'une quelconque des revendications précédentes, **caractérisée en ce que** la capsule est configurée pour examiner l'intestin grêle en tant qu'espace sous examen (5).

8. Capsule d'endoscope selon l'une quelconque des revendications précédentes 2 à 7, **caractérisée en ce que** la surface tissulaire (2) est alignée avec un plan focal (27) du moyen de détecteur optique de mouvement (8).

9. Capsule d'endoscope selon l'une quelconque des revendications précédentes, **caractérisée en ce que** les images enregistrées avec la caméra (7) sont stockées sur un stockage de données interne (15) de la capsule (4).

10. Capsule d'endoscope selon l'une quelconque des revendications précédentes, **caractérisée en ce qu'**un axe optique (31) de la caméra (7) et un axe optique (32) du détecteur optique de mouvement (8) sont tous deux orientés dans des directions différentes.

11. Capsule d'endoscope selon la revendication 10, dans laquelle l'axe optique (31) de la caméra (7) est de préférence parallèle à l'axe longitudinal (30) de la capsule et l'axe optique (32) du détecteur optique de mouvement (8) est de préférence perpendiculaire à l'axe longitudinal (30) de la capsule.

12. Capsule d'endoscope selon l'une quelconque des revendications 3 à 11, dans laquelle une fréquence de clichés du détecteur optique de mouvement (8) est d'au moins 30 clichés par seconde et de préférence entre 30 et 60 clichés par seconde.

13. Capsule d'endoscope selon l'une quelconque des revendications précédentes, dans laquelle la caméra (7) est activée chaque fois que la capsule a couvert une distance fixe prédéterminée après une image enregistrée précédemment.

14. Capsule d'endoscope selon l'une quelconque des revendications 3 à 13, dans laquelle le module capteur de mouvement ou traceur (12) a un DSP qui est adapté pour détecter des motifs sur les photographies prises par le capteur CMOS, pour évaluer comment ces motifs ont changé depuis la photographie précédente et pour déterminer si ou sur quelle distance la capsule s'est déplacée sur la base du changement des motifs sur une séquence d'images.
